(19) Europäisches Patentamt
European Patent Office
Office européen des brevets

(11) Veröffentlichungsnummer: **0 004 070**
**B1**

(12) **EUROPÄISCHE PATENTSCHRIFT**

(45) Veröffentlichungstag der Patentschrift: **19.05.82**

(51) Int. Cl.³: **C 07 D 263/12,**
C 07 D 263/04

(21) Anmeldenummer: **79100678.6**

(22) Anmeldetag: **07.03.79**

(54) **2-Methylen-3-(3-methyl-2-butenyl)-oxazolidine(II), Verfahren zu deren Herstellung, bei diesem Verfahren benötigte Zwischenprodukte und Verwendung von II zur Herstellung von 2-(2,2-Dimethyl-3-buten-1-yl)-2-oxazolinen(I).**

(30) Priorität: **15.03.78 DE 2811190**

(43) Veröffentlichungstag der Anmeldung:
**19.09.79 Patentblatt 79/19**

(45) Bekanntmachung des Hinweises auf die Patenterteilung:
**19.05.82 Patentblatt 82/20**

(84) Benannte Vertragsstaaten:
**BE CH DE FR GB IT NL**

(56) Entgegenhaltungen:
**JOURNAL OF ORGANIC CHEMISTRY, Vol. 39, Nr. 3, 8. Februar 1974, Washington, USA, R.E. IRELAND et al.: "The Claisen rearrangement of N-allylketene O, N-acetals", Seite 421—424.**

(73) Patentinhaber: **BAYER AG**
**Zentralbereich Patente, Marken und Lizenzen**
**D-5090 Leverkusen 1, Bayerwerk (DE)**

(72) Erfinder: **Jautelat, Manfred, Dr.**
**Müllersbaum 28**
**D-5093 Burscheid 1 (DE)**
Erfinder: **Arlt, Dieter, Dr.**
**Rybniker Strasse 2**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 070 B1

Courier Press, Leamington Spa, England.

2-Methylen-3-(3-methyl-2-butenyl)-oxazolidine(II), Verfahren zu deren Herstellung, bei diesem Verfahren benötigte Zwischenprodukte und Verwendung von II zur Herstellung von 2-(2,2-Dimethyl-3-buten-1-yl)-2-oxazolinen(I)

Die vorliegende Erfindung betrifft ein chemisch eigenartiges Verfahren zur Herstellung von 2-(2,2-Dimethyl-3-buten-1-yl)-2-oxazolinen. Diese können als Zwischenprodukte zur Herstellung von Insektiziden wie beispielsweise 2-(2,2-Dichlorvinyl)-3,3-dimethyl-cyclopropancarbonsäure-3-phenoxybenzylester verwendet werden.

2-(2,2-Dimethyl-3-buten-1-yl)-oxazoline sind Gegenstand einer anderen Anmeldung der Anmelderin. Sie können nach einem nicht zum Stand der Technik gehörenden Verfahren, das analog zu bekannten Verfahren verläuft, aus 3,3-Dimethyl-4-pentensäure und deren Derivate durch Umsetzung mit Ethanolaminen erhalten werden (vgl. DT—A—27 32 213.0). Da aber 3,3-Dimethyl-4-pentensäure und deren Derivate nur in mehrstufigen Syntheseverfahren zugänglich sind, ist dieser Herstellungsweg für 2-(2,2-Dimethyl-3-buten-1-yl)-oxazoline sehr aufwendig.

Aus J. Org. Chem. Vol 39, S. 421—424 war bekannt, daß 3-Methyl-2-butenyl-substituierte γ-Hydroxy-amide zu Dihydroxysäuren umlagern. Diese Reaktion verläuft jedoch aufgrund des 3-Methyl-2-butenyl-Substituenten in nur 13%iger Ausbeute.

1. Es wurden die 2-Methylen-3-(3-methyl-2-butenyl)-oxazolidine der allgemeinen Formel II

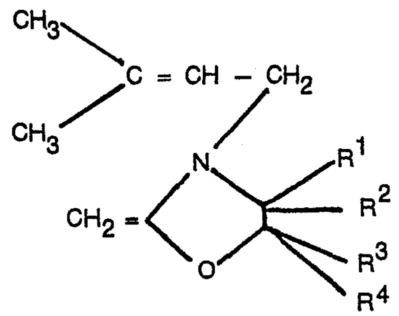

(II)

in welcher

$R^1$—$R^4$ unabhängig voneinander für Wasserstoff oder $C_{1-4}$-Alkyl stehen, gefunden.

2. Es wurde ferner ein Verfahren zur Herstellung der 2-Methylen-3-(3-methyl-2-butenyl)-oxazolidine der allgemeinen Formel II

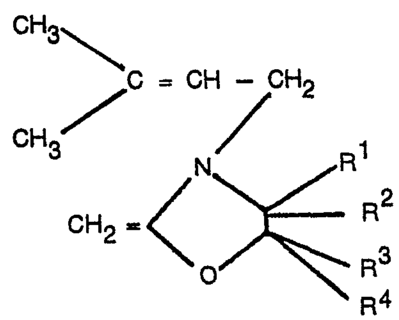

(II)

in welcher

$R^1$—$R^4$ für Wasserstoff oder $C_{1-4}$-Alkyl stehen, gefunden,

dadurch gekennzeichnet, daß man Oxazolidine der allgemeinen Formel III

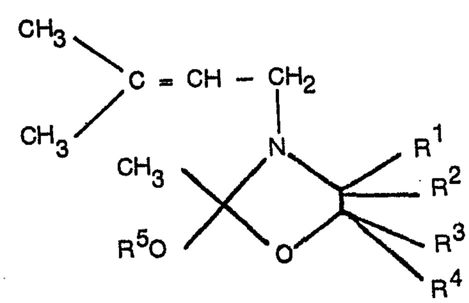

(III)

in welcher

$R^1$—$R^4$ die obengenannte Bedeutung haben und

$R^5$ für Alkyl oder Aryl steht,

in der Flüssig- oder Gasphase in Gegenwart oder Abwesenheit eines Verdünnungsmittels erhitzt.

3. Es wurde ferner die Verwendung von 2-Methylen-3-(3-methyl-2-butenyl)-oxazolidinen der allgemeinen Formel II

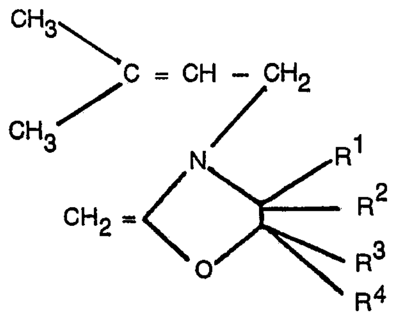

(II)

in welcher

$R^1$—$R^4$ für Wasserstoff oder $C_{1-4}$-Alkyl stehen,

zur Herstellung von 2-(2,2-Dimethyl-3-buten-1-yl)-2-oxazolinen der allgemeinen Formel I

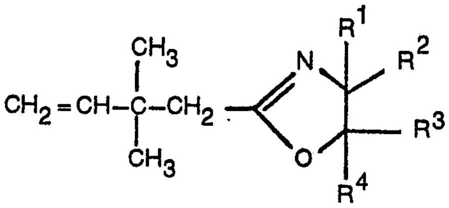

(I)

in welcher

$R^1$, $R^2$, $R^3$, $R^4$ unabhängig voneinander für Wasserstoff oder $C_{1-4}$-Alkyl stehen,

dadurch gekennzeichnet, daß man die Verbindungen der Formel II gegebenenfalls in Gegenwart eines Verdünnungsmittels erhitzt, gefunden.

4. Es wurden ferner die Oxazolidine der allgemeinen Formel III

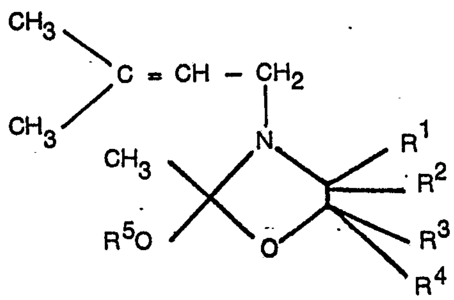

(III)

in welcher

$R^1$—$R^5$ die oben angegebene Bedeutung haben,

verwendet im Verfahren gemäß 2 (oben) gefunden.

5. Es wurde ferner ein Verfahren zur Herstellung von Oxazolidinen der allgemeinen Formel III

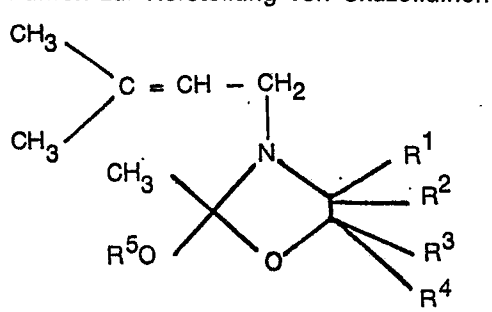

(III)

in welcher

$R^1$—$R^5$ die unter 2 (oben) angegebene Bedeutung haben,

verwendet im Verfahren gemäß 2 (oben), dadurch gekennzeichnet, daß man 2-Methyl-3-(3-methyl-2-butenyl)-2-oxazoliniumsalze der allgemeinen Formel IV

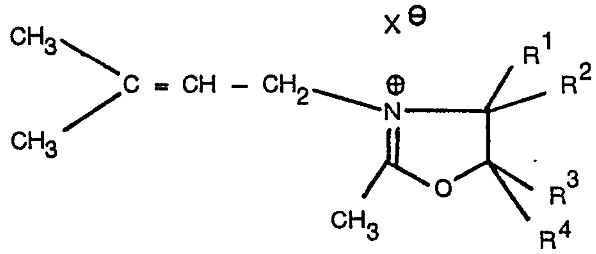

(IV)

in welcher
$R^1$, $R^2$, $R^3$ und $R^4$ die obige Bedeutung haben und
X für Halogen, Sulfonat oder Phosphat steht,
mit Alkoholaten der allgemeinen Formel V

$$R^5—O—M \qquad (V)$$

in welcher
M für ein Äquivalent eines Alkali- oder Erdalkalikations steht und
$R^5$ für Alkyl oder Aryl steht,
umsetzt, gefunden.

6. Es wurden ferner 3-Methyl-3-(3-methyl-2-butenyl)-2-oxazoliniumsalze der allgemeinen Formal IV

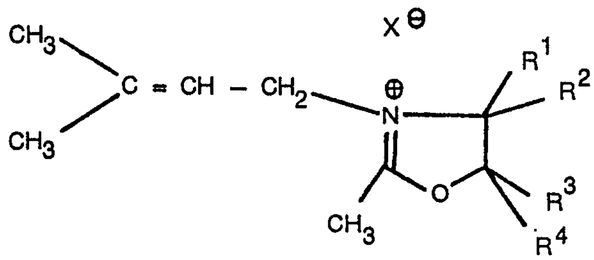

(IV)

in welcher
$R^1$, $R^2$, $R^3$ und $R^4$ die unter 2 (oben) angegebene Bedeutung haben und
X für Halogen, Sulfonat, Phosphat steht,
verwendet im Verfahren gemäß 5 (oben) gefunden.

7. Es wurde ferner ein Verfahren zur Herstellung von 2-Methyl-3-(3-methyl-2-butenyl-2-oxazoliniumsalzen der allgemeinen Formel IV

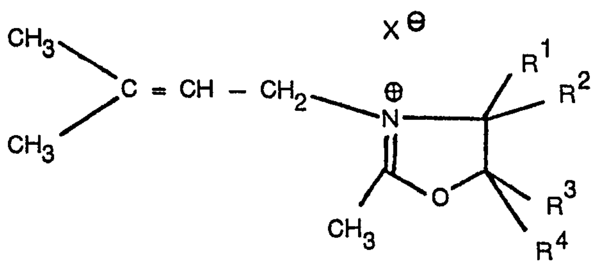

(IV)

in welcher
$R^1$, $R^2$, $R^3$ und $R^4$ die unter 2 (oben) angegebene Bedeutung haben und
X für Halogen, Sulfonat, Phosphat steht,
verwendet gemäß Verfahren 5 (oben) gefunden, dadurch gekennzeichnet, daß man 2-Methyloxazoline der allgemeinen Formel VI

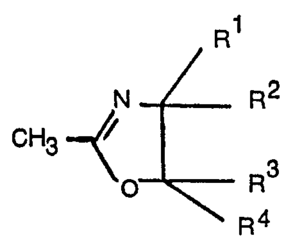

(VI)

in welcher
$R^1$—$R^4$ die obige Bedeutung haben,
mit 2-Methyl-2-buten-1-yl-Derivaten der allgemeinen Formel VII

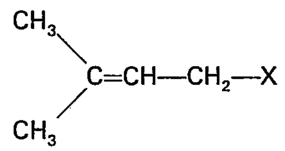
(VII)

in welcher

X die obige Bedeutung hat,

umsetzt.

Die Pyrolyse der Oxazolidine der Formel III führt zu den 2-Methylen-3-(3-methyl-2-butenyl)-oxazolidinen der Formel II die zu den 2-Oxazolinen der allgemeinen Formel I umlagern. Der selektive Verlauf dieser Reaktion war überraschend, da der Versuch diese Reaktion analog zu bekannten Reaktionen basenkatalysiert durchzuführen zu polymeren Produkten führt (vgl. Chem. Ber. *97*, 3081 (1964) und Liebigs Ann. Chem. *641*, 1 (1961)).

Es ist ferner überraschend, daß die Umsetzung der Oxazolidiniumsalze der Formel IV mit Alkoholaten der Formel V nicht unter Ringöffnung zu Acetamiden der Formel VIII

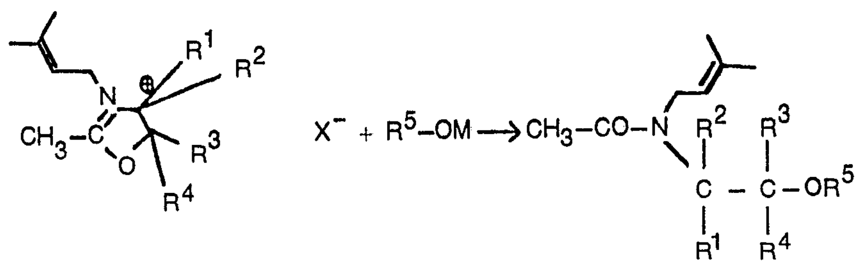
(VI)

wie es aufgrund ähnlicher Umsetzungen mit Pyridin zu vermuten war (J. Macrom. Sci. Chem. *1972*, 1631),

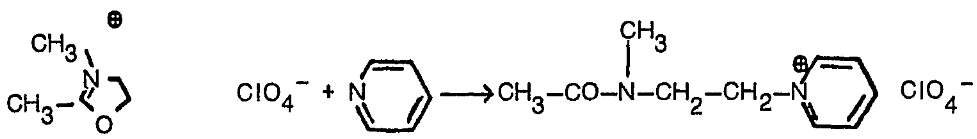

sondern in selektiver Weise unter Addition zu den bisher unbekannten 2-Alkoxy-2-methyl-3-(3-methyl-2-butenyl)-oxazolidinen führt.

Das Verfahren zur Herstellung der Oxazoline der Formel I weist, den Vorteil auf, daß die dabei verwendeten Ausgangsstoffe aus gut zugänglichen und preiswerten Stoffen wie z.B. 2-Methyloxazolin und Isopren hergestellt werden können.

Verwendet man 2-Methylen-3-(3-methyl-2-butenyl)-oxazolidin als Ausgangsstoff, kann der Reaktionsablauf durch folgendes Formelschema wiedergegeben werden:

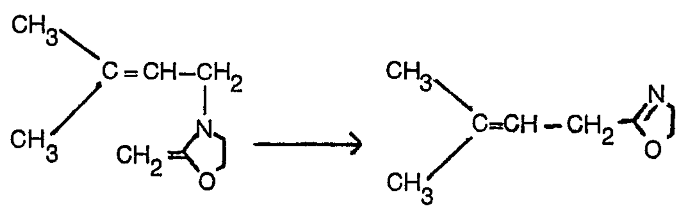

Verwendet man 2-Methoxy-2-methyl-(3-methyl-2-butenyl)-oxazolidin als Ausgangsstoff und führt die Reaktion durch, ohne 2-Methylen-3-(3-methyl-2-butenyl)-oxazolidin zu isolieren, so kann der Reaktionsablauf durch folgendes Formelschema wiedergegeben werden:

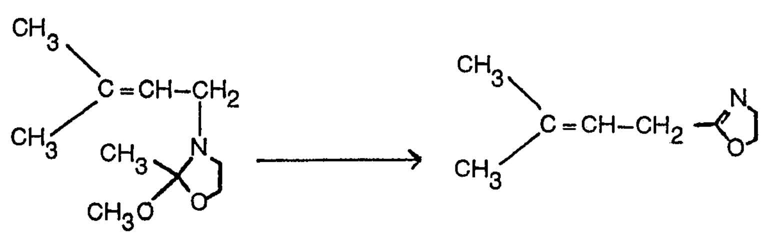

Die Herstellung von 2-Methoxy-2-methyl-(3-methyl-2-butenyl)-oxazolidin durch Umsetzung von 2-Methyl-3-(3-methyl-2-butenyl)-2-oxazoliniumbromid mit Natriummethylat läßt sich durch folgendes Formelschema wiedergegeben:

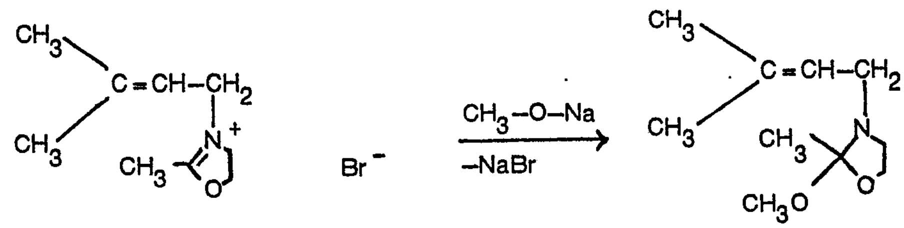


Die Herstellung von 2-Methyl-3-(3-methyl-2-butenyl)-2-oxazoliniumbromid aus 2-Methyl-2-oxazolin und 3-Methyl-2-butenylbromid läßt sich durch folgendes Formelschema wiedergeben:

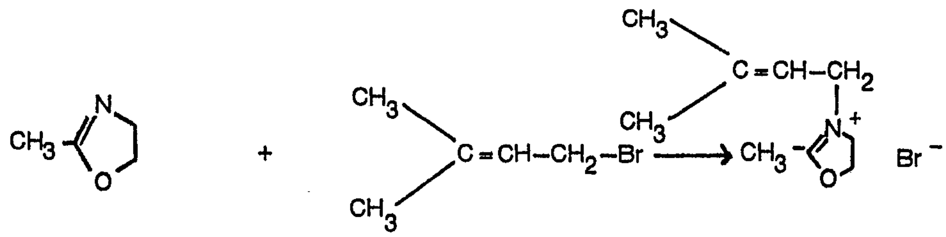

Verwendet man 2-Methyl-2-oxazolin und 3-Methyl-2-butenylbromit (Prenylbromid) als Ausgangsstoffe, so kann der Reaktionsablauf durch das folgende Formelschema wiedergegeben werden:

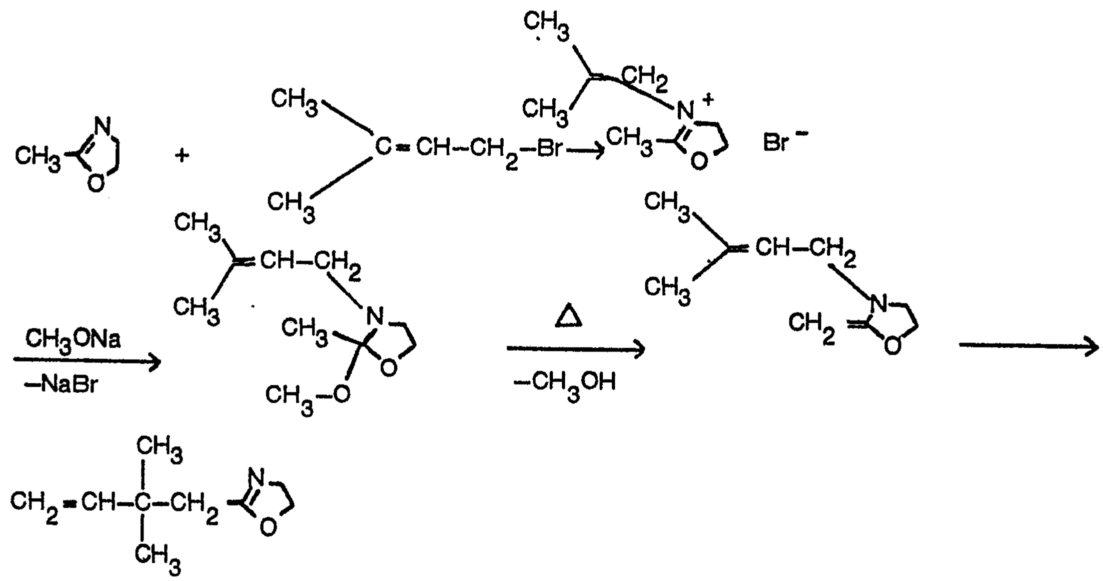


2-Methylen-3-(3-methyl-2-butenyl)-oxazolidine sind durch die Formel II allgemein definiert.

Als Beispiele für die erfindungsgemäß verwendbaren 2-Methylen-3-(3-methyl-2-butenyl)-oxazolidine seien die nach den obengenannten Reaktionen aus den folgenden 2-Methyl-2-oxazolinen erhältlichen genannt:

2-Methyl-2-oxalin, 2,4,4-Trimethyl-2-oxazolin, 2,5,5-Trimethyl-2-oxazolin und 2,4,4,5,5-Pentamethyl-2-oxazolin.

Die zur Herstellung der erfindungsgemäß verwendbaren 2-Methylen-3-(3-methyl-2-butenyl)-oxazolidine nach den obengenannten Reaktionen verwendbaren 3-Methyl-2-buten-1-yl-Derivate sind durch die Formel VII allgemein definiert. X steht vorzugsweise für Chlorid, Bromid, Jodid, Methansulfonat, p-Toluolsulfonat oder Benzolsulfonat.

Die ebenfalls zur Herstellung der erfindungsgemäß verwendbaren 2-Methylen-3-(methyl-2-butenyl)-oxazolidine nach den obengenannten Reaktionen verwendbaren Alkoholate sind durch die Formel V allgemein definiert. $R^5$ steht vorzugsweise für Alkylreste mit 1—4 C-Atomen oder Aryl wie Phenyl. M steht vorzugsweise für Natrium, Kalium, Magnesium, Calcium. Als Beispiel seien im einzelnen genannt: Natriummethylat, Natriumäthylat, Natriumpropylat, Natriumbutylat, Kaliummethylat, Magnesiummethylat oder Calciummethylat.

Die Herstellung der Oxazoliniumsalze der Formel VI kann sowohl in Gegenwart als auch in Abwesenheit eines inerten Verdünnungsmittels im Temperaturbereich von 0—80°C, vorzugsweise 20—60°C, erfolgen. Als Verdünnungsmittel kommen Kohlenwasserstoffe wie Benzin, Toluol, Chloroform, Methylenchlorid, 1,2-Dichlorethan, Ether wie Diäthylether, Dioxan, Alkohole wie Methanol, Ethanol, Butanol oder Acetonitril oder DMF infrage.

Die Umsetzung der Oxazoliniumsalze der Formel IV mit Alkoholaten der Formel V kann sowohl mit isolierten Oxazoliniumsalzen in einem separatem Verfahrensschritt als auch im Anschluß an die Herstellung der Oxazoliniumsalze in einer Eintopf-Reaktion durchgeführt werden. Als Lösungsmittel kommen Alkohole wie Methanol, Ethanol, Butanol, aber auch inerte Verdünnungsmittel wie Kohlenwasserstoffe, beispielsweise Toluol, Methylenchlorid oder Chlorbenzol, Ether, beispielsweise Diethylether, THF, Dioxan oder Dimethyoxyethan, als auch DMSO, DMF, HMPT oder Acetonitril infrage. Die Reaktion wird im Temperaturbereich von 0°—100°C, vorzugsweise 15°C—60°C, ausgeführt.

Die Herstellung der 2-(2,2-Dimethyl-3-buten-1-yl)-2-oxazoline wird nun bevorzugt ohne Isolierung der als Zwischenstufe auftretenden 2-Methylen-3-(3-methyl-2-butenyl)-oxazolidine durch Pyrolyse der Oxazolidine der Formel III durchgeführt.

Die Pyrolyse der Oxazolidine der Formel III kann einmal in der Gasphase erfolgen, indem die Dämpfe in einem Inertgasstrom wie Stickstoff unter Druck oder unter Normaldruck durch ein geheiztes Rohr, das mit inerten Wärmüberträgern wie Glaskugeln gefüllt ist, geleitet und anschließend kondensiert werden. Oder man verdampft das Oxazolidin unter vermindertem Druck und führt diese Dämpfe durch ein beheiztes Rohr zur Spaltung. Zum anderen ist die Pyrolyse auch in der Flüssigphase möglich. Man erhitzt eine Lösung der Oxazolidine in einem inerten Verdünnungsmittel oder tropft die Lösung auf einen Wärmeüberträger bei geeigneten Temperaturen. Die Pyrolyse und Umlagerung kann in einem größeren Temperaturbereich variiert werden. Im allgemeinen arbeitet man bei Temperaturen von 200—500°C, vorzugsweise bei 300—400°C. Als Verdünnungsmittel eignen sich alle inerten Lösungsmittel wie Kohlenwasserstoffe, beispielsweise Toluol, Xylol, Cyclohexan, Decalin, Chinolin oder Ether, wie Dioxan, Diphenylether.

In den einzelnen Verfahrensstufen werden die Reaktionspartner im äquimolaren Verhältnis eingesetzt. Doch Abweichungen davon, wobei eine Komponente im Über- oder Unterschuß eingesetzt wird, sind möglich.

Die folgenden Beispiele erläutern das erfindungsgemäße Verfahren.

Beispiel 1

8,5 g (0,1 Mol) 2-Methyloxazolin und 14,9 g (0,1 Mol) 3-Methyl-2-butenylbromid (Prenylbromid) werden 2 Stunden bei 20—25°C gerührt, wobei in exothermer Reaktion ein kristallines, hygroskopisches 2-Methyl-3-(3-methyl-2-butenyl)-2-oxazoliniumbromid entsteht.

NMR ($CDCl_3$): $\delta$ 1,7—1,9 (m, 6H); 2,7 (s, 3H); 4,2—4,4 (m, 4H); 5,15 (d, 2H), 5,5 (t, 1H).

Beispiel 2

23,4 g (0,1 Mol) 2-Methyl-3-(3-methyl-2-butenyl)-2-oxazolinium-bromid werden mit 22 ml einer 5 n Natriummethylatlösung 1 Stunde bei 40°C gerührt, vom ausgeschiedenen Natriumchlorid abgetrennt, mit abs. Ether gewaschen und einrotiert. Die zurückbleibende Flüssigkeit destilliert bei $Kp_{20}$ 96—98°C; 15,2 g (82 mmol, 82%) 2-Methoxy-2-methyl-3-(3-methyl-2-butenyl)-oxazolidin.

NMR ($CDCl_3$): $\delta$ 1,35 (s, 3H); 1,6—1,75 (m, 6H); 2,8—3,25 (m, 4H); 3,2 (s, 3H); 3,8—4,1 (m, 2H); 5,25 (t, 1H).

Beispiel 3

18,5 g (0,1 Mol) 2-Methoxy-2-methyl-3-(3-methyl-2-butenyl)-oxazolidin werden bei einem Druck von 20 mbar destilliert, die Dämpfe durch ein mit Glaskugeln gefülltes, 400°C heißes Rohr geführt und anschließend kondensiert. Im Kondensat von 16,8 g findet man bei einem Umsatz von 73% 2-(2,2-Dimethyl-3-butenyl)-2-oxazolin in einer Ausbeute von 86%. Durch fraktionierte Destillation wird das reine Produkt ($Kp_{14}$ 78—80°C) isoliert.

NmR ($CDCl_3$): 1,1 (s, 6H); 2,3 (s, 2H); 3,7—4,4 (m, 4H); 4,8—6,2 (3H, $—CH=CH_2$).

Beispiel 4

9,25 g (50 mmol) 2-Methoxy-2-methyl-3-(3-methyl-2-butenyl)-oxazolidin werden als 10% Lösung in Toluol im Stickstoffstrom auf 350°C heiße Glaskugeln in einem beheizten Rohr getropft und anschließend kondensiert. Das Kondensat wird vom Lösungsmittel befreit und bei $Kp_{18}$ 80—82° destilliert. Man isoliert 6,0 g (39 mmol, 78%) 2-(2,2-Dimethyl-3-butenyl)-2-oxazolin, das mit obigem Produkt (Beispiel 3) identisch ist.

Beispiel 5

8,5 g (0,1 Mol) 2-Methyloxazolin und 14,9 g (0,1 Mol) 3-Methyl-2-butenylbromid werden in 50 ml Acetonitril 2 Stunden bei 20° bis 25°C gerührt und nach Zusatz von 20 ml einer 5 n Natriummethylatlösung weitere 3 Stunden bei Raumtemperatur gerührt. Die Lösung wird filtriert und nach dem Abziehen des Lösungsmittels destilliert. Man gewinnt 15,6 g (84 mmol, 84% 2-Methoxy-2-methyl-3-(3-methyl-2-butenyl)-oxazolidin vom $Kp_{20}$ 96—99°C.

Beispiel 6

Die gleiche Umsetzung wie im Beispiel 5 in 50 ml Methylenchlorid ergibt das obige Oxazolidin in einer Ausbeute von 79%.

Beispiel 7

8,5 g (0,1 mol) 2-Methyloxazolin und 14,9 g (0,1 mol) Prenylbromid werden in 50 ml n-Hexan 1 Std. bei 40° gerührt, wobei eine Suspension entsteht, die nach dem Zusatz von 0,11 mol einer Natriumethylatlösung in Ethanol nochmal 1Std. bei 40° gerührt wird. Die Lösung wird abgesaugt, einrotiert und an der Ölpumpe destilliert. Bei $Kp._{0,1}$ 67° werden 12,3 g (0,062 mol, 62%) 2-Ethoxy-2-methyl-3-(3-methyl-2-butenyl)-oxazolidin isoliert.

Beispiel 8

10 g (50 mmol) 2-Ethoxy-2-methyl-3-(3-methyl-2-butenyl)-oxazolidin werden im Stickstoffstrom auf 325° heiße Glaskugeln in einem beheizten Rohr getropft und in einem angeschlossenen Kondensator niedergeschlagen. Man entfernt den Alkohol im Vakuum und destilliert das zurückbleibende 2-(2,2-Dimethyl-3-butenyl)-2-oxazolin bei $Kp._{17}$ 76—78° (6,5 g; 42,5 mmol, 85%).

Beispiel 9

14,9 g (0,1 mol) Prenylbromid und 8,5 g (0,1 mol) 2-Methyloxazolin werden in 50 ml Methylenchlorid 2Std. bei 25° gerührt, mit 0,11 mol einer Natriumbutylatlösung in n-Butanol 1Std. bei 40° behandelt und in üblicher Weise aufgearbeitet. Durch Destillation bei $Kp_{0,3}$ 78—80° gewinnt man 13,4 g (59 mmol, 59%) 2-Butoxy-2-methyl-3-(3-methyl-2-butenyl)-oxazolidin.

Beispiel 10

Eine Pyrolyse von 11,4 g (50 mmol) 2-Butoxy-2-methyl-3-(3-methyl-2-butenyl)-oxazolidin bei 400° gemäß Beispiel 8 führt zu 2-(2,2-Dimethyl-3-butenyl)-2-oxazolin in 70% Ausbeute.

Beispiel 11

30 g (0,162 mol) 2-Methoxy-2-methyl-3-(3-methyl-2-butenyl)-oxazolidin ergeben bei der Pyrolysé im Stickstoffstrom ohne Verdünnungsmittel bei 325° 88% 2-(2,2-Dimethyl-3-butenyl)-2-oxazolin.

**Patentansprüche**

1. 2-Methylen-3-(3-methyl-2-butenyl)-oxazolidin der allgemeinen Formel II

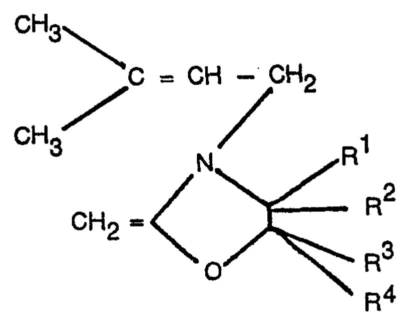

(II)

in welcher

$R^1$—$R^4$ unabhängig voneinander für Wasserstoff oder $C_{1-4}$-Alkyl stehen.

2. Verfahren zur Herstellung von 3-Methylen-3-(3-methyl-2-butenyl)-oxazolidin der allgemeinen Formel II

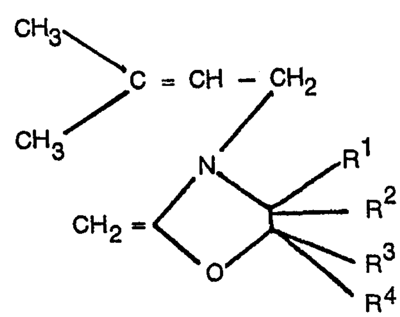

(II)

in welcher

$R^1$—$R^4$ für Wasserstoff oder $C_{1-4}$-Alkyl stehen,

dadurch gekennzeichnet, daß man Oxazolidine der allgemeinen Formel III

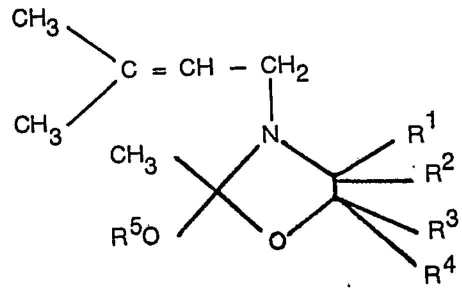

(III)

in welcher
$R^1$—$R^4$ die obengenannte Bedeutung haben und
$R^5$ für Alkyl oder Aryl steht,
in der Flüssig- oder Gasphase in Gegenwart oder Abwesenheit eines Verdünnungsmittels erhitzt.

3. Verwendung von 2-Methylen-3-(3-methyl-2-butenyl)-oxazolidin der allgemeinen Formel II

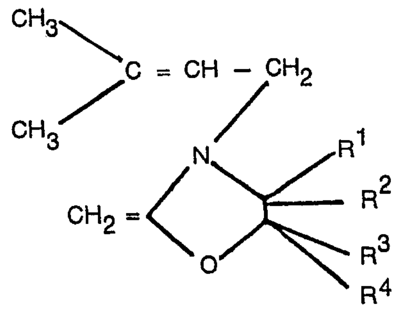

(II) ,

in welcher
$R^1$—$R^4$ für Wasserstoff oder $C_{1-4}$-Alkyl stehen,
zur Herstellung von 2-(2,2-Dimethyl-3-buten-1-yl)-2-oxazolinen der allgemeinen Formel I

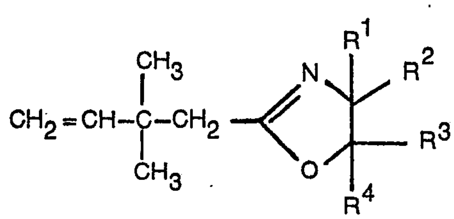

(I)

in welcher
$R^1$, $R^2$, $R^3$, $R^4$ unabhängig voneinander für Wasserstoff oder $C_{1-4}$-Alkyl stehen,
dadurch gekennzeichnet, daß man die Verbindungen der Formel II gegebenenfalls in Gegenwart eines Verdünnungsmittels erhitzt.

4. Oxazolidine der allgemeinen Formel III

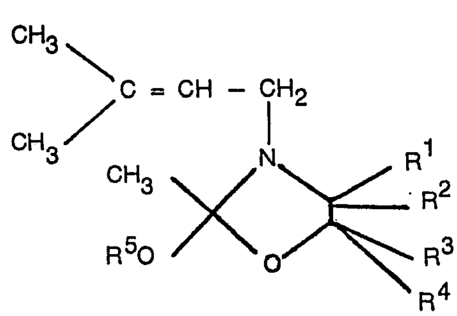

(III)

in welcher
$R^1$—$R^5$ die in Anspruch 2 angegebene Bedeutung haben,
verwendet im Verfahren gemäß Anspruch 2.

5. Verfahren zur Herstellung von Oxazolidinen der allgemeinen Formel III

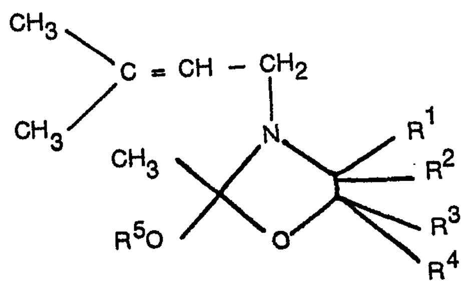

(III)

in welcher
$R^1$—$R^5$ die in Anspruch 2 angegebene Bedeutung haben,
verwendet im Verfahren gemäß Anspruch 2, dadurch gekennzeichnet, daß man 2-Methyl-3-(3-methyl-2-butenyl)-2-oxazoliniumsalze der allgemeinen Formel IV

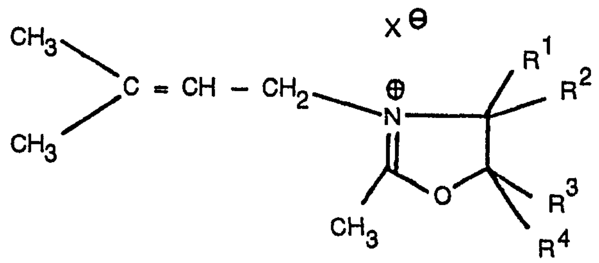

(IV)

in welcher
$R^1$, $R^2$, $R^3$ und $R^4$ die obige Bedeutung haben und
X für Halogen, Sulfonat oder Phosphat steht,
mit Alkoholaten der allgemeinen Formel V

$$R^5—O—M \qquad (V)$$

in welcher
M für ein Äquivalent eines Alkali- oder Erdalkalikations steht und
$R^5$ für Alkyl oder Aryl steht,
umsetzt.

6. 2-Methyl-3-(3-methyl-2-butenyl)-2-oxazoliniumsalze der allgemeinen Formal IV

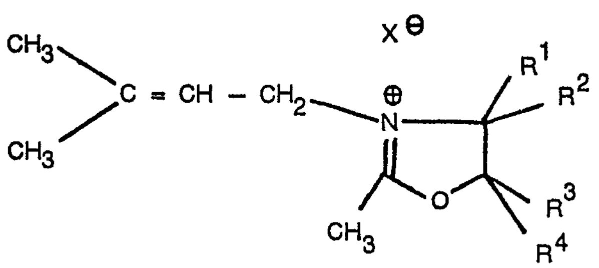

(IV)

in welcher
$R^1$, $R^2$, $R^3$ und $R^4$ die in Anspruch 2 angegebene Bedeutung haben und
X für Halogen, Sulfonat, Phosphat steht,
verwendet im Verfahren gemäß Anspruch 5.

7. Verfahren zur Herstellung von 2-Methyl-3-(3-methyl-2-butenyl-2-oxazoliniumsalzen der allgemeinen Formel IV

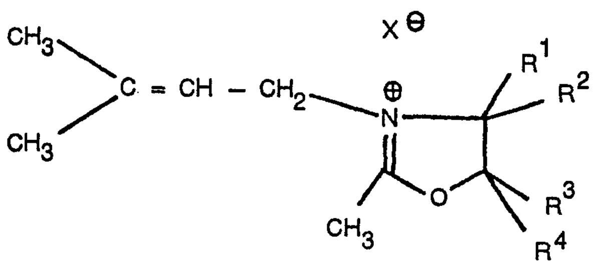

(IV)

in welcher
$R^1$, $R^2$, $R^3$ und $R^4$ die in Anspruch 2 angegebene Bedeutung haben und
X für Halogen, Sulfonat, Phosphat steht,

verwendet gemäß Anspruch 5, dadurch gekennzeichnet, daß man 2-Methyloxazoline der allgemeinen Formel VI

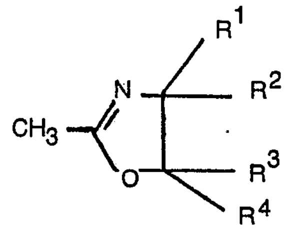

(VI)

in welcher

$R^1$—$R^4$ die obige Bedeutung haben,

mit 2-Methyl-2-buten-1-yl-Derivaten der allgemeinen Formel VII

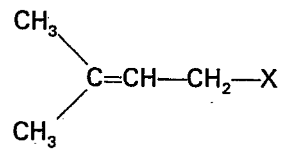

(VII)

in welcher

X die obige Bedeutung hat,

umsetzt.

**Revendications**

1. 2-méthylène-3-(3-méthyl-2-buténiyl)-oxazolidine de formule générale II:

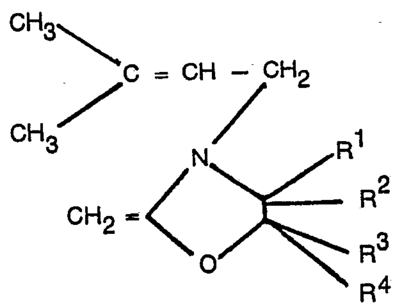

(II)

dans laquelle

$R^1$—$R^4$ représentent chacun indépendamment l'un de l'autre un atome d'hydrogène ou un groupe alkyle contenant 1 à 4 atomes de carbone.

2. Procédé de préparation de 2-méthylène-3-(3-méthyl-2-buténiyl)-oxazolidine de formule générale II:

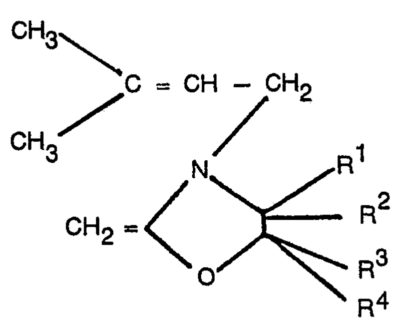

(II)

dans laquelle

$R^1$—$R^4$ représentent chacun un atome d'hydrogène ou un groupe alkyle contenant 1 à 4 atomes de carbone,

caractérisé en ce qu'on chauffe des oxazolidines de formule générale III:

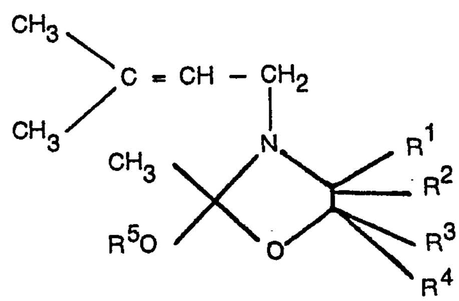

(II)

dans laquelle

$R^1$—$R^4$ ont les significations indiquées ci-dessus, et

$R^5$ représente un groupe alkyle ou un groupe aryle,

en phase liquide ou en phase gazeuse, en présence ou en absence d'un diluant.

3. Utilisation de 2-méthylène-3-(3-méthyl-2-buténуl)-oxazolidine de formule générale II:

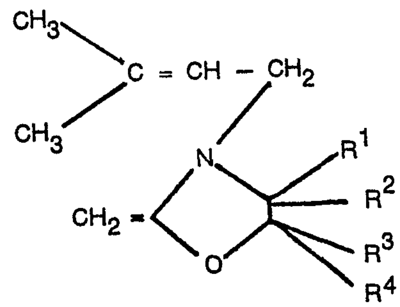

(III)

dans laquelle

$R^1$—$R^4$ représentent chacun un atome d'hydrogène ou un groupe alkyle contenant 1 à 4 atomes de carbone,

pour la préparation de 2-(2,2-diméthyl-3-butén-1-yl)-2-oxazolines de formule générale I:

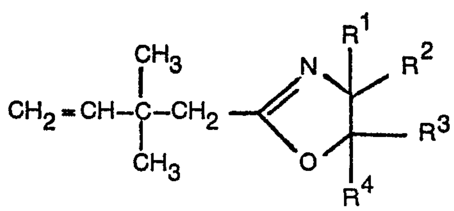

(I)

dans laquelle

$R^1$, $R^2$, $R^3$ et $R^4$ représentent chacun indépendamment l'un de l'autre un atome d'hydrogène ou un groupe alkyle contenant 1 à 4 atomes de carbone,

caractérisé en ce qu'on chauffe les composés de formule II éventuellement en présence d'un diluant.

4. Oxazolidines de formule générale III:

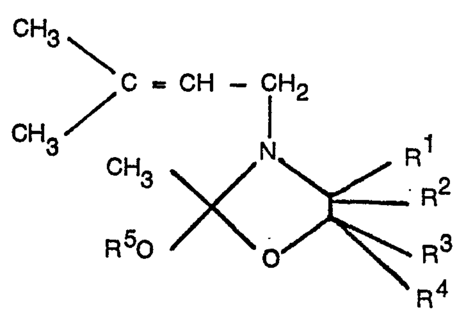

(III)

dans laquelle

$R^1$—$R^5$ ont les significations indiquées dans la revendication 2,

ces oxazolidines étant utilisées dans le procédé suivant la revendication 2.

5. Procédé de préparation d'oxazolidines de formule générale III:

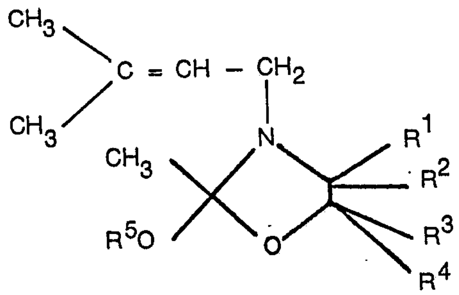

(III)

dans laquelle

$R^1$—$R^5$ ont les significations indiquées dans la revendication 2,

ces oxazolidines étant utilisées dans le procédé suivant la revendication 2, caractérisé en ce qu'on fait réagir des sels de 2-méthyl-3-(3-méthyl-2-butényl)-2-oxazolinium de formule générale IV:

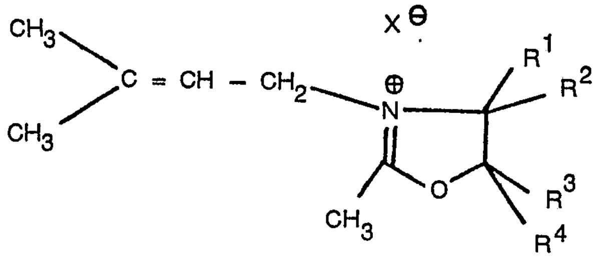

(IV)

dans laquelle

$R^1$, $R^2$, $R^3$ et $R^4$ ont les significations indiquées ci-dessus, et

X représente un atome d'halogène, un groupe sulfonate ou un groupe phosphate,

avec des alcoolates de formule générale V:

$$R^5—O—M \quad (V)$$

dans laquelle

M représente un équivalent d'un cation alcalin ou alcalino-terreux, et

$R^5$ représente un groupe alkyle ou un groupe aryle.

6. Sels de 2-méthyl-3-(3-méthyl-2-buténył)-2-oxazolium de formule générale IV:

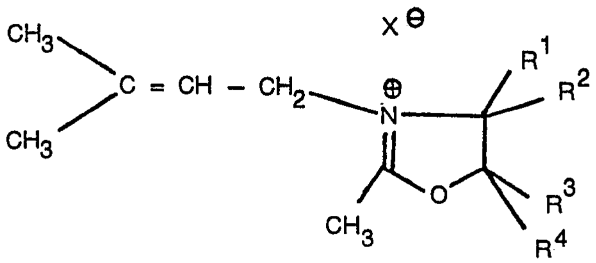

(IV)

dans laquelle

$R^1$, $R^2$, $R^3$ et $R^4$ ont les significations indiquées dans la revendication 2, et

X représente un atome d'halogène, un groupe sulfonate ou un groupe phosphate,

ces sels étant utilisés dans le procédé suivant la revendication 5.

7. Procédé de préparation de sels de 2-méthyl-3-(3-méthyl-2-buténył)-2-oxazolinium de formule générale IV:

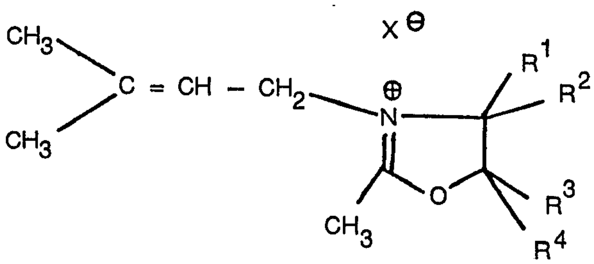

(IV)

dans laquelle

$R^1$, $R^2$, $R^3$ et $R^4$ ont les significations indiquées dans la revendication 2, et X représente un atome d'halogène, un groupe sulfonate ou un groupe phosphate, ces sels étant utilisés suivant la revendication 5, caractérisé en ce qu'on fait réagir des 2-méthyl-oxazolines de formule générale VI:

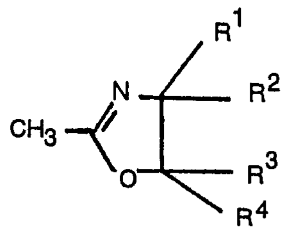

(VI)

dans laquelle $R^1$—$R^4$ ont les significations indiquées ci-dessus, avec des dérivés 3-méthyl-2-butén-1-yle de formule générale VII:

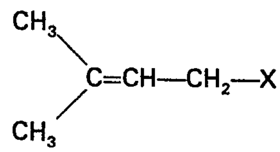

(VII)

dans laquelle X a la signification indiquée ci-dessus.

**Claims**

1. 2-Methylene-3-(3-methyl-2-butenyl)-oxazolidine of the general formula II

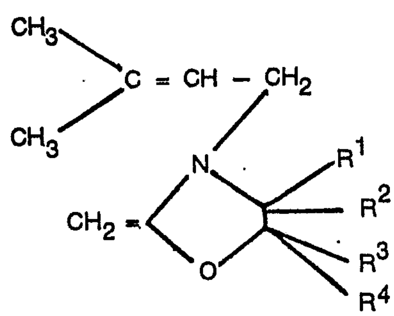

(II)

in which $R^1$—$R^4$, independently of one another, represent hydrogen or $C_{1-4}$-alkyl.

2. Process for the preparation of 2-methylene-3-(3-methyl-2-butenyl)-oxazolidine of the general formula II

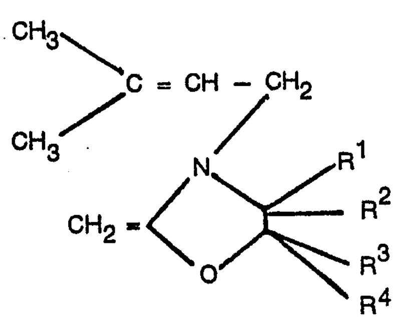

(II)

in which $R^1$—$R^4$ represent hydrogen or $C_{1-4}$-alkyl, characterised in that oxazolidines of the general formula III .

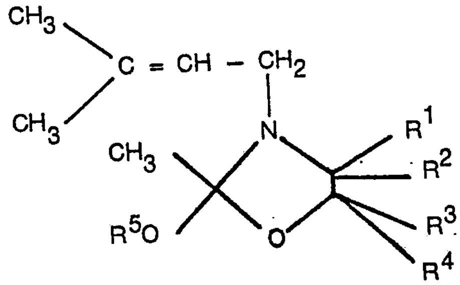

(III)

in which
$R^1$—$R^4$ have the above-mentioned meaning and
$R^5$ represent alkyl or aryl,
are heated in the liquid phase or gas phase in the presence or absence of a diluent.

2. Use of 2-methylene-3-(3-methyl-2-butenyl)-oxazolidine of the general formula II

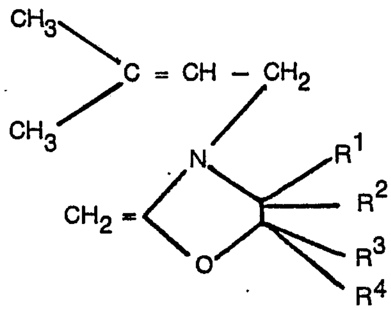

(II)

in which
$R^1$—$R^4$ represent hydrogen or $C_{1-4}$-alkyl,
for the preparation of 2-(2,2-dimethyl-3-buten-1-yl)-2-oxazolines of the general formula I

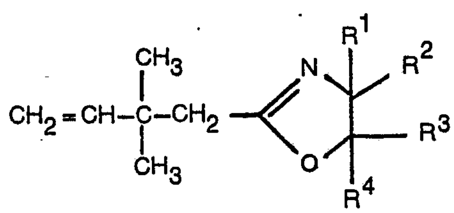

(I)

in which
$R^1$, $R^2$, $R^3$ and $R^4$, independently of one another represent hydrogen or $C_{1-4}$-alkyl,
characterised in that the compounds of the formula II are heated optionally in the presence of a diluent.

4. Oxazolidines of the general formula III

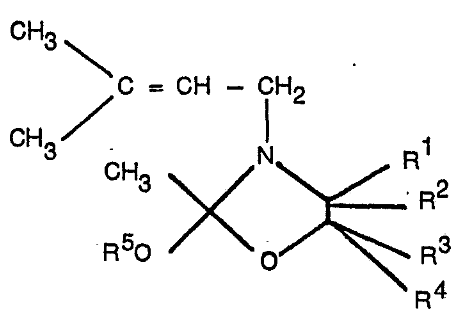

(III)

in which
$R^1$—$R^5$ have the meaning given in Claim 2,
used in the process according to Claim 2.

5. Process for the preparation of oxazolidines of the general formula III

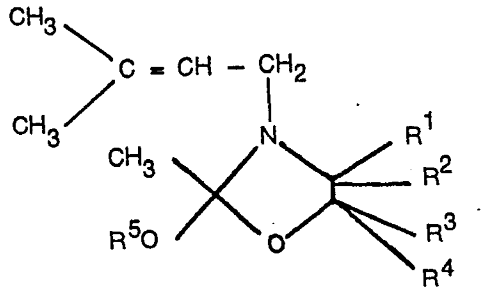

(III)

in which

$R^1$—$R^5$ have the meaning given in Claim 2,

used in the process according to Claim 2, characterised in that 2-methyl-3-(3-methyl-2-butenyl)-2-oxazolinium salts of the general formula IV

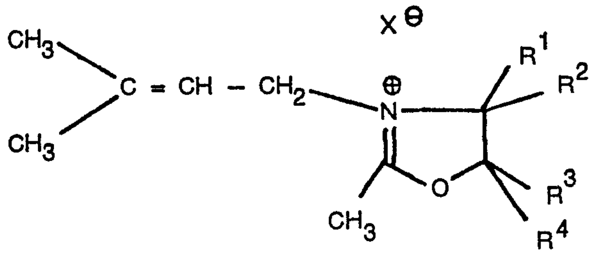

(IV)

in which

$R^1$, $R^2$, $R^3$ and $R^4$ have the above meaning and

X represents halogen, sulphonate or phosphate

are reacted with alcoholates of the general formula V

$$R^5—O—M \qquad (V)$$

in which

M represents one equivalent of an alkali metal cation or alkaline earth metal cation and

$R^5$ represents alkyl or aryl.

6. 2-Methyl-3-(3-methyl-2-butenyl)-2-oxazolinium salts of the general formula IV

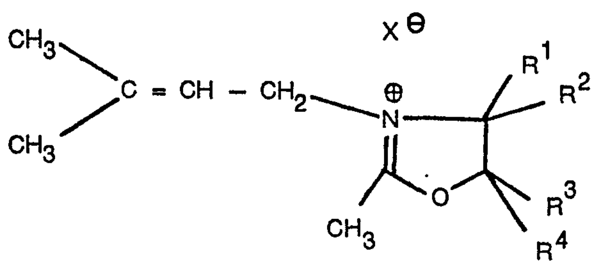

(IV)

in which

$R^1$, $R^2$, $R^3$ and $R^4$ have the meaning given in Claim 2 and

X represents halogen, sulphonate or phosphate,

used in the process according to Claim 5.

7. Process for the preparation of 2-methyl-3-(3-methyl-2-butenyl-2-oxazolinium salts of the general formula IV

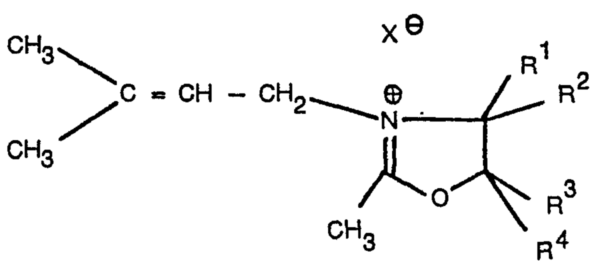

(IV)

in which

$R^1$, $R^2$, $R^3$ and $R^4$ have the meaning given in Claim 2 and
X represents halogen, sulphonate or phosphate,
used according to Claim 5, characterised in that 2-methyloxazolines of the general formula VI

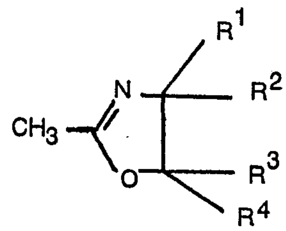

in which
$R^1$—$R^4$ have the above meaning,
are reacted with 3-methyl-2-buten-1-yl derivatives of the general formula VII

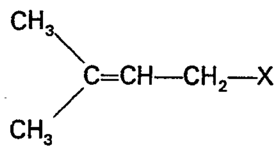

(VII)

in which
X has the above meaning.